# EUROPÄISCHE PATENTSCHRIFT

(11) **EP 0 885 607 B1**
(45) Veröffentlichungstag und Bekanntmachung des Hinweises auf die Patenterteilung: **11.02.2004**
(21) Anmeldenummer: 98110850.9
(22) Anmeldetag: 10.06.1998
(51) Int. Cl.: A61K 7/50, A61K 7/48, A61K 7/06

(54) **Neutrale avivierende und emulgierende Zubereitung zur Haar- oder Faserbehandlung**
Neutral brightening and emulsifying composition for treating hair or fibres
Composition neutre et émulsifiante pour l'avivage des cheveux ou des fibres

(30) Priorität: 19.06.1997 DE 19726046
(43) Veröffentlichungstag der Anmeldung: 23.12.1998
(73) Patentinhaber: Henkel Kommanditgesellschaft auf Aktien, 40589 Düsseldorf-Holthausen (DE)
(72) Erfinder: Hollenberg, Detlef, Dr., 40699 Erkrath (DE); Goddinger, Dieter, Dr., 25436 Tornesch (DE); Priebe, Christian, 42489 Wülfrath (DE)

(56) Entgegenhaltungen:
- DE-A- 19 540 853

## Beschreibung

Die Erfindung betrifft Zubereitungen zur Faserbehandlung, bevorzugt zur Behandlung von keratinischen Fasern und besonders bevorzugt zur Behandlung von menschlichen Haaren, enthaltend eine spezielle Wirkstoffkombination aus mindestens einem Alkylamidoamin und mindestens einer, mindestens eine primäre, sekundäre oder tertiäre Aminogruppe enthaltenden Polycarbonsäure.

Menschliches Haar wird heute in vielfältiger Weise mit haarkosmetischen Zubereitungen behandelt. Dazu gehören etwa die Reinigung der Haare sowie das Bleichen, Färben und Verformen der Haare mit Färbemitteln, Stylingpräparaten und Wellmitteln. Sowohl die Reinigung der Haare mit Shampoos als auch die dekorative Gestaltung der Frisur, beispielsweise durch Färben oder Dauerwellen, sind Eingriffe, welche die natürliche Struktur und die Eigenschaften der Haare beeinflussen. Nahezu alle Arten der Haarbehandlung können mit unerwünschten Beeinträchtigungen der Haarstruktur einhergehen. Diese Beeinträchtigungen äußern sich unter anderem in einer verschlechterten Kämmbarkeit der Haare in nassem bzw. trockenem Zustand, einer verstärkten elektrostatischen Aufladung, einer verstärkten Sprödigkeit, einer verringerten Höchstreißkraft und Reißdehnung der Haare sowie in einem verschlechterten äußeren Erscheinungsbild der Frisur.

Es hat daher nicht an Ansätzen gefehlt, die Haare mit entsprechenden Wirkstoffen nachzubehandeln, um die erwähnten Begleiterscheinungen in ihrem Ausmaß zu mildern oder zu beseitigen. In der Regel werden dazu kationische Tenside herangezogen, häufig in Kombination mit weiteren Hilfsstoffen. Eine solche Nachbehandlung wird beispielsweise in Form einer Spülung oder Haarkur angewandt, die als Wirkstoff beispielsweise quartäre Ammoniumsalze oder spezielle Polymere beinhaltet. Dieses aus dem Stand der Technik weithin bekannte Vorgehen ist jedoch bislang in mehrfacher Hinsicht unbefriedigend.

Die üblicherweise bislang als kationische Tenside eingesetzten quartären Ammoniumverbindungen sind häufig nur unbefriedigend biologisch abbaubar, so daß ihr Einsatz aus ökologischen Gründen möglichst verringert oder ganz vermieden werden sollte. Weiterhin können die Ammoniumverbindungen bei empfindlichen Personen in einzelnen Fällen zu Irritationen auf der Haut führen.

Da die bislang in Zubereitungen zur Faserbehandlung eingesetzten kationischen Tenside auch eine erhebliche emulgierende Wirkung aufweisen, wurden sie zum Teil auch gezielt als Emulgatoren, alleine oder in Kombination mit weiteren Emulgatoren, in Mitteln zur Behandlung von Fasern oder zur Behandlung der Haut eingesetzt.

In der Regel muß davon ausgegangen werden, daß die kationischen Tenside der sie enthaltenden Zubereitung einen sauren Charakter verleihen. Meist sind solche kationischen Tenside sogar nur in saurer Umgebung einsetzbar, d.h. im neutralen pH-Bereich (pH-Werte von etwa 6,5 bis etwa 7,5) verlieren sie teilweise oder ganz ihre gewünschte Wirkung. Dies führt entweder zu einer Eigenschaftsverschlechterung des die kationischen Tenside enthaltenden Produkts, oder es hat den Einsatz höherer Mengen an kationischem Tensid zur Folge, was zu höheren Kosten führt und ökologisch unvorteilhaft ist.

Haarbehandlungsmittel mit saurem pH-Wert üben zudem noch eine unangenehme Wirkung auf die Augenschleimhaut aus. Häufig gelangt beim Gebrauch der Haarbehandlungsmittel ein geringer Anteil in die Augen, was zu einem unangenehmen "Brennen" der Augenschleimhaut des Betroffenen führt. Diese nachteilige Eigenschaft ist weitgehend auf einen solchen niedrigen pH-Wert der eingesetzten Haarbehandlungsmittel zurückzuführen.

Es bestand daher Bedarf nach Zubereitungen zur Faserbehandlung, die bei pH-Werten im neutralen Bereich (etwa 6,5 bis etwa 7,5) avivierend wirken, gute Emulgatorwirkung zeigen und zudem gute biologische Abbaubarkeit, geringes Irritationspotential und geringe Ökotoxizität aufweisen.

Die nicht vorveröffentlichte deutsche Patentanmeldung mit dem Aktenzeichen 97 100 586.3 (DE-A-196 02 242) betrifft avivierende Zubereitungen, die Alkylamidoamine und quaternäre Esterverbindungen enthalten. Zur Einstellung des pH-Werts der Zubereitungen werden Genußsäuren, wie Essigsäure, Milchsäure, Weinsäure, Citronensäure, Äpfelsäure, Ascorbinsäure und Gluconsäure eingesetzt. Die beispielhaft offenbarten Zubereitungen weisen einen pH-Wert von 3,5 auf.

Es wurde nun gefunden, daß der Einsatz von mindestens einem Alkylamidoamin zusammen mit mindestens einer, mindestens eine primäre, sekundäre oder tertiäre Aminogruppe enthaltenden Polycarbonsäure die Formulierung von Zubereitungen erlaubt, die auch bei neutralen pH-Werten eine gute Avivagewirkung in Verbindung mit guten Emulsionseigenschaften zeigen. Die so erhältlichen Zubereitungen zeigen außerdem ein geringes Irritationspotential für Haut und Augen, geringe Ökotoxizität und gute biologische Abbaubarkeit.

Gegenstand der Erfindung ist daher eine wäßrige Zubereitung zur Haut- oder Faserbehandlung, enthaltend mindestens ein Alkylamidoamin der allgemeinen Formel I 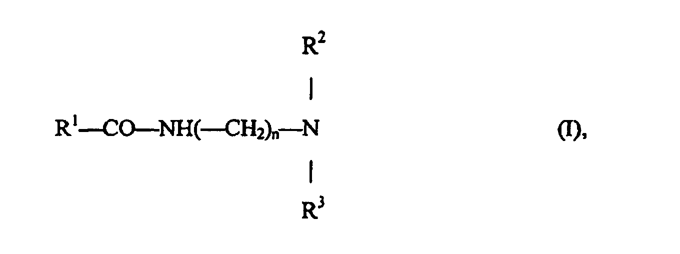 worin R¹―CO für einen aliphatischen, linearen oder verzweigten, bis zu 3 Doppelbindungen aufweisenden C₆-C₂₂-Acylrest und n für Werte von 1 bis 10 steht und R² und R³ unabhängig voneinander für lineare oder verzweigte C₁-C₂₂-Alkylreste stehen, wobei zusätzlich mindestens eine, mindestens eine primäre, sekundäre oder tertiäre Aminogruppe aufweisende Polycarbonsäure enthalten ist und das molare Verhältnis von Alkylamidoaminmolekülen zu freien Säuregruppen zwischen 0,9 und 1,1 liegt.

Die erfindungsgemäße Zubereitung enthält mindestens ein Alkylamidoamin der allgemeinen Formel I, das der Zubereitung eine faseravivierende Wirkung verleiht und emulgierend wirkt. Die Verbindungen aus der Gruppe der Alkylamidoamine mit der allgemeinen Formel I werden im folgenden Text der Einfachheit halber als *"Komponente A*"bezeichnet.

In der allgemeinen Formel I steht R¹―CO für einen aliphatischen, linearen oder verzweigten Acylrest mit 6 bis 22 Kohlenstoffatomen, der 0, 1, 2 oder 3 Doppelbindungen aufweist und in der Regel aus einer natürlich vorkommenden oder synthetisch hergestellten Fettsäure stammt. Bevorzugt steht R¹―CO für einen aliphatischen, linearen oder verzweigten Acylrest mit 14 bis 20 Kohlenstoffatomen, besonders bevorzugt mit 18 Kohlenstoffatomen.

R² und R³ stellen gleiche oder unterschiedliche Alkylreste dar, die gegebenenfalls funktionelle Gruppen wie Hydroxygruppen, Estergruppen, Amine, Amide oder sonstige polare Substituenten tragen können, wobei jedoch in der Regel unsubstituierte Kohlenwasserstoffreste, die gegebenenfalls eine oder mehrere Verzweigungen aufweisen können, bevorzugt sind. Besonders bevorzugt sind lineare, kurzkettige Kohlenwasserstoffreste mit 1 bis 6 C-Atomen, wobei Ethyl- und Methylreste erfindungsgemäß ganz besonders bevorzugt sind.

Die Variable n steht für einen Wert von 1 bis etwa 10, wobei Werte von 2 bis 7, insbesondere von 2 bis 4, bevorzugt sind.

Alkylamidoamine werden üblicherweise durch Amidierung natürlicher oder synthetischer Fettsäuren und Fettsäureschnitte mit Dialkylaminoaminen hergestellt. Typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Besonders bevorzugt sind die aus Cocosöl oder Palmöl erhältlichen Fettsäureschnitte, ganz besonders bevorzugt ist der Einsatz von Stearinsäure.

Wird die Komponente A unter Verwendung von Fettsäureschnitten hergestellt, so ergibt sich daraus zwangsläufig, daß nicht nur eine Sorte Alkyl- amidoamin der allgemeinen Formel I vorliegt, sondern ein Gemisch aus mehreren Alkylamidoaminen der allgemeinen Formel I. Die im Gemisch vorliegenden Alkylamidoamine unterscheiden sich durch den jeweiligen Rest R¹―CO, entsprechend der Carbonsäurezusammensetzung des verwendeten Fettsäureschnitts. Die erfindungsgemäßen Zusammensetzungen können entsprechend auch ein Gemisch aus mehreren Verbindungen der allgemeinen Formel I enthalten, wobei solche Gemische ebenfalls unter *"Komponente A"* zusammengefaßt werden.

Ein Gemisch aus mehreren Alkylamidoaminen, wie es im Sinne der Erfindung als Komponente A einsetzbar ist, muß jedoch nicht zwangsläufig aus dem Einsatz von Fettsäureschnitten bei der Herstellung des Alkylamidoamins resultieren. Ebensogut kann durch Mischen verschiedener reiner Alkylamidoamine eine Mischung mit einer gewünschten Zusammensetzung erzeugt werden, oder es kann beispielsweise in einem aus Fettsäureschnitten hergestellten Gemisch von Alkylamidoaminen ein bestimmtes Alkylamidoamin durch nachträgliche Zugabe der reinen Form des jeweiligen Alkylamidoamins angereichert werden.

Die erfindungsgemäße Zubereitung enthält die Komponente A in der Regel in einem Anteil von etwa 0,01 bis etwa 5 Gew.-%. Bevorzugt enthält die Zubereitung etwa 0,05 bis 3 Gew.-%, besonders bevorzugt etwa 0,1 bis 2 Gew.-% und insbesondere bevorzugt etwa 0,5 bis etwa 1,5 Gew.-% der Komponente A.

Zusammen mit der Komponente A wird in der erfindungsgemäßen Zubereitung mindestens eine, mindestens eine primäre, sekundäre oder tertiäre Aminogruppe aufweisende Polycarbonsäure eingesetzt. Die Polycarbonsäure oder ein ebenso einsetzbares Gemisch aus mehreren solcher Polycarbonsäuren wird im folgenden Text der Einfachheit halber als *"Komponente B"* bezeichnet.

Grundsätzlich sind als Komponente B alle Polycarbonsäuren einsetzbar, die wenigstens eine primäre, sekundäre oder tertiäre Aminogruppe tragen. In der Praxis hat es sich jedoch bewährt, solche Verbindungen einzusetzen, deren Molekulargewicht etwa 500, vorzugsweise etwa 400 und besonders bevorzugt etwa 350 nicht überschreitet

Insbesondere handelt es sich bei diesen speziellen Polycarbonsäuren der Komponente B um Derivate der Di-, Tri- oder Tetracarbonsäuren. Beispiele für erfindungsgemäß einsetzbare Polycarbonsäuren sind Nitrilotriessigsäure (NTA), Ethylendiamintetraessigsäure (EDTA), β-Alanindiessigsäure (ADA), Asparaginsäure (ASP), Glutaminsäure (GLU) oder Cystin.

Ebenfalls geeignet sind die Polycarbonsäuren heterocyclischer, stickstoffhaltiger Verbindungen, beispielsweise die Polycarbonsäuren des Pyridins, des Pyrrolidins oder des Pyrrolidons.

Die Komponente B wird in der Regel in einem Verhältnis (bezogen auf freie Säuregruppen) von etwa 0,9 bis etwa 1,1 zur Komponente A eingesetzt. Vorzugsweise wird die Komponente A dabei gerade neutralisiert, d.h., das Verhältnis von Säuregruppen zu Alkylamidoaminmolekülen oder zum Gemisch aus Alkylamidoaminmolekülen ist stöchiometrisch (etwa 1).

Neben der Komponente A und der Komponente B enthalten die erfindungsgemäßen Zubereitungen Wasser in unterschiedlichen Mengen. Der Wasseranteil beträgt in der Regel zwischen etwa 99,99 und 0,1 Gew.-%, bezogen auf die gesamte Zubereitung.

Eine Rezeptur enthaltend etwa 10 bis 85 Gew.-% Komponente A, etwa 1 bis 15 Gew.-% Komponente B und gegebenenfalls Wasser ad 100 Gew.-%, kann als Basisformulierung zur Herstellung einer Vielzahl von Produkten aus dem Körperpflegebereich dienen, beispielsweise zur Formulierung von Produkten zur Haut- oder zur Haarbehandlung. Die Basisrezepturen zeigen eine gute emulgierende Wirkung, und die erfindungsgemäße Kombination von Komponente A und Komponente B läßt sich als alleiniger Emulgator zur Herstellung einer Vielzahl von Emulsionen einsetzen.

Insbesondere läßt sich die erfindungsgemäße Kombination aus Komponente A und Komponente B in wäßriger Zubereitung in folgenden Körperpflegeprodukten einsetzen: Haarspülungen, Haarkuren, Haarfärbemittel, Haarspitzenfluids, Wellmittel, Hautcremes.

Die erfindungsgemäße Zubereitung kann in den genannten Körperpflegemitteln zum einen eine avivierende Funktion erfüllen, zum anderen kann Sie auch zusätzlich zu weiteren bereits avivierend wirkenden Verbindungen eingesetzt werden. Auch wenn eine avivierende Wirkung im Körperpflegemittel nicht erforderlich ist, können die erfindungsgemäßen Zubereitungen als emulgierende Komponente verwendet werden. In jedem Fall zeigt die erfindungsgemäße Zubereitung eine gute emulgierende Wirkung im pHneutralen Bereich bei gleichzeitig guter biologischer Abbaubarkeit.

Neben Komponente A und Komponente B können in der erfindungsgemäßen Zubereitung noch weitere Stoffe vorliegen, welche der Zubereitung die jeweiligen Eigenschaften zum endgültigen Gebrauch als Körperpflegemittel verleihen. Beispiele für solche Stoffe sind kationische Polymere, anionische Polymere, amphotere Polymere, kationische Tenside, kationische Silikonöle, quaternäre Esterverbindungen, anionische Tenside, nichtionische Tenside, Fettalkohole, Vitamine oder Vitaminderivate, Färbemittel, Lösemittel und Konservierungsmittel sowie Gemische aus zwei oder mehr der genannten Komponenten.

Für spezielle Anwendungen der erfindungsgemäßen Zubereitungen, beispielsweise als Färbemittel oder als Wellotion können zusätzlich zu einer oder mehreren der aufgezählten Komponenten noch Kuppler, Entwickler, direktziehende Farbstoffe, Enzyme, Reduktionsmittel oder Fixiermittel (Oxidationsmittel) sowie Gemische aus zwei oder mehr der aufgezählten Substanzen in der erfindungsgemäßen Zubereitung vorliegen. Zusätzlich können gegebenenfalls noch weitere, in der Körperpflege oder der Kosmetik übliche Zusatzstoffe vorhanden sein.

Für die Haarbehandlung besonders gut geeignet sind Zubereitungen, die neben Komponente A und Komponente B zusätzlich noch eine polymere Verbindung enthalten. Bevorzugt sind Polymere aus der Gruppe der kationischen, anionischen und amphoteren Polymeren.

Geeignete kationische Polymere enthalten üblicherweise ein quartäres Stickstoffatom, beispielsweise in Form einer Ammoniumgruppe. Bevorzugte kationische Polymere sind beispielsweise
- quaternisierte Cellulose-Derivate, wie sie unter den Bezeichnungen Celquat® und Polymer JR® im Handel erhältlich sind; die Verbindungen Celquat® H 100, Celquat® L 200 und Polymer JR®400 sind bevorzugte quaternierte Cellulose-Derivate;
- Polysiloxane mit quaternären Gruppen,
- polymere Dimethyldiallylammoniumsalze und deren Copolymere mit Estern und Amiden von Acrylsäure und Methacrylsäure; die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere;
- Copolymere des Vinylpyrrolidons mit quaternierten Derivaten des Dialkylaminoacrylats- und -methacrylats, wie beispielsweise mit Di-ethylsulfat quaternierte Vinylpyrrolidon-Dimethylaminomethacrylat-Copolymere; solche Verbindungen sind unter den Bezeichnungen Gafquat®734 und Gafquat®755 im Handel erhältlich;
- Vinylpyrrolidon-Methoimidazoliniumchlorid-Copolymere, wie sie unter der Bezeichnung Luviquat® angeboten werden,
- quaternierter Polyvinylalkohol sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Beispiele für erfindungsgemäß geeignete anionische Polymere sind:
- Polyacryl- und Polymethacrylsäuren, deren Salze, deren Copolymere mit Acrylsäure- und Methacrylsäureestem und -amiden und deren Derivate, die durch Kreuzvernetzung mit polyfunktionellen Agentien erhalten werden; Verbindungen dieser Art sind beispielsweise unter den Bezeichnungen Carbopol®934, Carbopol®934P, Carbopol®940, Carbopol®950, Carbopol®980 und als Carbopol®EDT-Typen (Hersteller: alle BF Goodrich) sowie PNC®400 (Hersteller: 3V Sigma) erhältlich;
- Polyoxycarbonsäuren, wie Polyketo- und Polyaldehydocarbonsäuren und deren Salze, beispielsweise POC-HS 5060 und POC-AS 5060 (Degussa, Frankfurt),
- Polymere und Copolymere der Crotonsäure mit Estern und Amiden der Acryl- und der Methacrylsäure, oder Vinylacetat-Crotonsäure- und Vinylacetat-Vinylpropionat-Crotonsäure-Copolymere; Verbindungen die-ser Art sind als Luviset®CA-66 und Luviset®CAP (Hersteller: beide BASF, Ludwigshafen) erhältlich.

Unter dem Oberbegriff Ampho-Polymere sind amphotere Polymere, d.h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und -COO⁻- und/oder -SO₃⁻-Gruppen enthalten, und solche Polymeren zusammengefaßt, die -COOH- und/oder SO₃H-Gruppen und quartäre Ammoniumgruppen enthalten.

Ein Beispiel für ein erfindungsgemäß einsetzbares Amphopolymer ist das unter der Bezeichnung Amphomer erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte Ampho-Polymere sind solche Polymerisate, die sich im wesentlichen zusammensetzen aus
(a) Monomeren mit quartären Ammoniumgruppen der allgemeinen Formel II,

   [R⁴-CH=CR⁵-CO-Z(-CₙH₂ₙ)-N⁽⁺⁾R⁶R⁷R⁸]A⁽⁻⁾ (II),

   in der R⁴ und R⁵ unabhängig voneinander stehen für Wasserstoff oder eine Methylgruppe und R⁶, R⁷ und R⁸ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel III

   R⁹-CH=CR¹⁰-COOH (III),

   in denen R⁹ und R¹⁰ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der DE-A 39 29 973 Bezug genommen. Ganz besonders bevorzugt sind solche Polymerisate, bei denen Monomere des Typs (a) eingesetzt werden, bei denen R⁶, R⁷ und R⁸ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Bevorzugt enthalten die erfindungsgemäßen Zubereitungen die kationischen, anionischen oder amphoteren Polymere in Mengen von 0,1 bis 5 Gew.-%, bezogen auf die gesamte Zubereitung.

Insbesondere wenn die erfindungsgemäßen Zubereitungen als Haarspülungen verwendet werden, ist die Auswahl von Tensiden aus der Gruppe der kationischen Tenside bevorzugt.

Beispiele, für die in den erfindungsgemäßen Haarbehandlungsmitteln verwendbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning®929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls sehr gut biologisch abbaubare kationische Tenside sind quaternäre Esterverbindungen, sogenannte *"Esterquats"*.

Mit *"Esterquats"* werden bekannte Stoffe bezeichnet, die man nach den einschlägigen Methoden der präparativen organischen Chemie erhalten kann. In diesem Zusammenhang sei auf die WO 91/01295 verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Übersichten zu diesem Thema sind beispielsweise von R. Puchta et al. in *Tens.Surf.Det*., 30, 186 (1993), M. Brock in *Tens.Surf.Det*., 30, 394 (1993) und R. Lagennan et al. in *J.Am.Oil.Chem.Soc*., 71, 97 (1994) erschienen.

Ein Beispiel für Esterquats sind die quaternierten Fettsäuretriethanol-aminestersalze der allgemeinen Formel IV 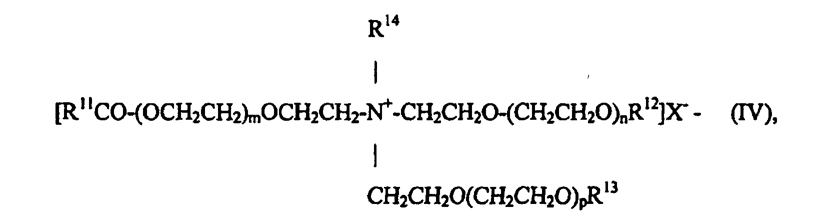 in der R¹¹CO für einen Acylrest mit 6 bis 22 Kohlenstoffatomen, R¹² und R¹³ unabhängig voneinander für Wasserstoff oder R¹¹CO, R¹⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X⁻ für Halogenid, Alkylsulfat oder Alkylphosphat steht.

Typische Beispiele für quaternierten Fettsäuretriethanolaminestersalze, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielweise bei der Druckspaltung natürlicher Fette und Öle anfallen.

Vorzugsweise werden technische C₁₂₋₁₈-Kokosfettsäuren und insbesondere teil-gehärtete C₁₆₋₁₈-Talg- bzw. Palmfettsäuren sowie Elaidinsäurereiche C₁₆₋₁₈-Fettsäureschnitte eingesetzt.

Zur Herstellung der quaternierten Fettsäuretriethanolaminestersalze der allgemeinen Formel IV können die Fettsäuren und das Triethanolamin im mola-ren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten quaternierten Fettsäuretriethanolaminestersalze der allgemeinen Formel IV stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab.

Aus anwendungstechnischer Sicht haben sich quaternierte Fettsäure-triethanolaminestersalze der allgemeinen Formel IV als besonders vorteilhaft erwiesen, wenn R¹¹CO für einen Acylrest mit 16 bis 18 Kohlenstoffatomen, R¹² für R¹¹CO, R¹³ für Wasserstoff, R¹⁴ für eine Methylgruppe, m, n und p für 0 und X⁻ für Methylsulfat steht.

Neben den quaternierten Fettsäuretriethanolaminestersalzen der allgemeinen Formel IV kommen als Esterquats ferner auch quaternierte Estersalze von Fettsäuren mit Diethanolalkylaminen der Formel V in Betracht 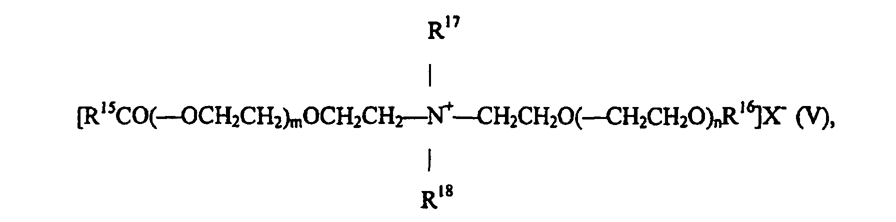 in der R¹⁵CO für einen Acylrest mit 6 bis 22 Kohlenstoffatomen, R¹⁶ für Wasserstoff oder R¹⁵CO, R¹⁷ und R¹⁸ unabhängig voneinander für Alkylreste mit 1 bis 4 Kohlenstoffatomen, m und n in Summe für 0 oder Zahlen von 1 bis 12 und X⁻ für Halogenid, Alkylsulfat oder Alkylphosphat steht.

Als weitere Gruppe geeigneter Esterquats sind schließlich die quatemierten Estersalze von Fettsäuren mit 1,2-Dihydroxypropyldialkylaminen der Formel VI zu nennen 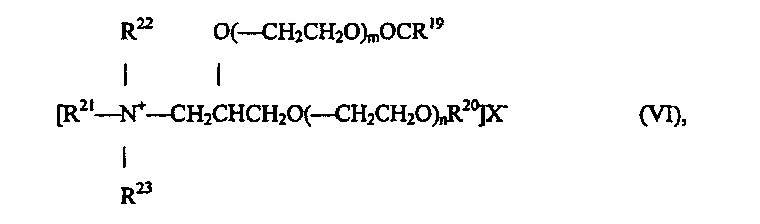 in der R¹⁹CO für einen Acylrest mit 6 bis 22 Kohlenstoffatomen, R²⁰ für Wasserstoff oder R¹⁹CO, R²¹, R²² und R²³ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für IV genannten Beispiele auch für die Esterquats der Formeln V und VI. Üblicherweise gelangen die Esterquats in Form 50 bis 90 Gew.-%iger alkoholischer Lösungen in den Handel, die bei Bedarf problemlos mit Wasser verdünnt werden können.

Die anhand der Formeln IV bis VI beschriebenen Substanzen stellen naturgemäß Einzelstoffe dar, wobei für den erfindungsgemäßen Einsatz auch Gemische dieser Stoffe infrage kommen können. Ebenso möglich ist der Einsatz der technischen Stoffgemische, wie sie aufgrund der Herstellungsverfahren in wechselnder Zusammensetzung erhältlich sind.

Ebenfalls als kationische Tenside in den erfindungsgemäßen Zubereitungen einsetzbar sind quarternäre Zuckerderivate.

Ein Beispiel für ein als kationisches Tensid einsetzbares quaternäres Zuckerderivat stellt das Handelsprodukt Glucquat® 100 dar, gemäß CTFA-Nomenklatur ein *"Lauryl Methyl Gluceth-10 Hydroxypropyl Dimonium Chloride"*.

Besonders bevorzugte kationische Tenside sind Esterquats und quaternäre Zuckerderivate.

Werden die erfindungsgemäßen Zubereitungen in Shampoos eingesetzt, so enthalten diese bevorzugt Tenside aus der Gruppe der anionischen Tenside.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine Wasserlöslichkeit verleihende anionische Gruppe, wie mindestens eine Carboxylat-, Sulfat, Sulfonat- oder Phosphat-Gruppe, und mindestens eine Kohlenwasserstoffgruppe, vorzugsweise mindestens eine lipophile Alkylgruppe mit etwa 10 bis etwa 22 C-Atomen. Zusätzlich können im Molekül Glykol- oder Polyglykolether-Gruppen, Estergruppen, Ether-, Amid- sowie Hydroxylgruppen enthalten sein. Beispiele für geeignete anionische Tenside sind:
- lineare Fettsäuren mit 10 bis 22 C-Atomen (Seifen),
- Ethercarbonsäuren der Formel R²⁴-O-(CH₂-CH₂O)ₓ-CH₂-COOH, in der R²⁴ eine lineare Alkylgruppe mit 10 bis 22 C-Atomen und x = 0 oder 1 bis 16 ist,
- Amidethercarboxylate der Formel [R²⁵-NH(-CH₂-CH₂-O)ₙ-CH₂-COO]-ₘA, in der R²⁵ für einen linearen oder verzweigten, gesättigten oder ungesättigten Acylrest mit 2 bis 29 C-Atomen, n für ganze Zahlen von 1 bis 10, m für die Zahlen 1 oder 2 und A für ein Kation aus der Gruppe der Alkali- oder Erdalkalimetalle steht,
- Acylsarcoside mit 10 bis 18 C-Atomen in der Acylgruppe, Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R²⁶(CH₂-CH₂O)ₓ-OSO₃H, in der R²⁶ eine, bevorzugt lineare, Alkylgruppe mit 10 bis 18 C-Atomen und x = 0 oder 1 bis 12 ist,
- sulfatierte Hydroxyalkylpolyethylen- und/oder Hydroxyalkylenpolypropylenglykolether gemäß der DE-A 37 23 354,
- Sulfonate ungesättigter Fettsäuren mit 12 bis 24 C-Atomen und 1 bis 6 Doppelbindungen gemäß der DE-A 39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,

jeweils in Form der Alkali-, Erdalkali oder Ammoniumsalze, vorzugsweise in Form ihrer Natrium-, Kalium-, Magnesium- und Ammonium- sowie der Mono-, Di- und Trialkanolammoniumsalze mit 2 oder 3 C-Atomen in der Alkanolgruppe.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

In den erfindungsgemäßen Mitteln können gegebenenfalls auch zwitterionische, amphotere oder nichtionogene Tenside enthalten sein. Als zwitterionische Tenside werden solche oberflächenaktiven Verbindungen bezeichnet, die im Molekül mindestens eine quartäre Ammoniumgruppe und eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Nichtionische Tenside enthalten als hydrophile Gruppe z. B. eine Polyolgruppe, eine Polyalkylenglykolethergruppe oder eine Kombination aus Polyol- und Polyglykolethergruppen. Solche Verbindungen sind beispielsweise:
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und/oder gehärtetes Rizinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit einer unterschiedlichen, vom jeweiligen Rohstoff abhängigen Zahl von Kohlenstoffatomen in der Alkylkette erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

In einer weiteren Ausführungsform ist es bevorzugt, als nichtionische Tenside Alkylpolyglykoside der allgemeinen Formel R²⁷O(-Z)ₓ auszuwählen. In den so bezeichneten Verbindungen enthält der Alkylrest R²⁷ 6 bis 22 Kohlenstoffatome und kann sowohl linear als auch verzweigt sein. Bevorzugt sind primäre lineare oder in 2-Stellung methylverzweigte Alkylreste. Solche Alkylreste R²⁷ sind beispielsweise 1-Octyl-, 1-Decyl-, 1-Lauryl-, 1-Myristyl-, 1-Cetyl- und 1-Stearylreste. Besonders bevorzugt sind 1-Octyl-, 1-Decyl-, 1-Lauryl- oder 1-Myristylreste. Bei Verwendung sogenannter *"OxoAlkohole"* als Ausgangsstoffe überwiegen Verbindungen mit einer ungeraden Anzahl von Kohlenstoffatomen in der Alkylkette.

Die in den erfindungsgemäßen Zubereitungen verwendbaren Alkylpolyglykoside können beispielsweise nur einen bestimmten Alkylrest R²⁷ enthalten. Üblicherweise werden die Alkylpolyglycoside aber ausgehend von natürlichen Fetten und Ölen oder Mineralölen hergestellt. In diesem Fall liegen als Alkylreste R²⁷ Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R²⁷
- im wesentlichen für C₈- und C₁₀-Alkylgruppen,
- im wesentlichen für C₁₂- und C₁₄-Alkylgruppen,
- im wesentlichen für C₈- bis C₁₆-Alkylgruppen oder
- im wesentlichen für C₁₂- bis C₁₆-Alkylgruppen steht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylpolyglykoside, bei denen x 1,1 bis 1,4 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Vorzugsweise enthalten die erfindungsgemäß verwendeten Zubereitungen die kationischen, zwitterionischen, anionischen und/oder nichtionischen Tenside in Mengen von etwa 0,5 bis etwa 40 Gew.-%, insbesondere von etwa 0,5 bis etwa 20 Gew.-% und besonders bevorzugt von etwa 1 bis etwa 5 Gew.%, bezogen auf die gesamte Zubereitung.

Die erfindungsgemäßen Zubereitungen liegen üblicherweise in Form von wäßrigen oder wäßrig-alkoholischen Lösungen oder Dispersionen vor. Bevorzugt sind dabei Formulierungen auf Wasserbasis sowie Formulierungen, die weniger als etwa 15 Gew.-% bezogen auf die gesamte Zubereitung, Alkohol, insbesondere Ethanol, enthalten. Für spezielle Anwendungen können aber auch Formulierungen auf Alkoholbasis, insbesondere auf Ethanolbasis, bevorzugt sein.

Die erfindungsgemäßen Zubereitungen können neben der zwingend vorliegenden Kombination aus Komponente A und Komponente B zusätzlich oder anstatt einer oder mehrerer der bereits aufgezählten, weiteren Verbindungen ein Vitamin und/oder ein Vitaminderivat enthalten. Grundsätzlich sind natürliche, synthetische, wasserlösliche oder öllösliche Vitamine und Vitaminderivate geeignet.

Besonders geeignete Vitamine und Vitaminderivate sind beispielsweise Vitamin A, Vitamin E, Vitamin E-Acetat, Vitamin E-Nicotinat, Vitamin F, Vitamin B₃, Vitamin B₆, Nicotinsäureamid, Vitamin H, Vitamin C, Vitamin B₅ und seine Derivate, insbesondere Panthenol, Panthotensäure, Calciumpanthotenat, Panthotenylethylether, Panthenylhydroxypropylsteardimoniumchlorid (Panthequat®, Innovachem), Panthetine und Panthenyltriacetat. Selbstverständlich sind analoge Derivate wie beispielsweise Panthotenylpropylether, Panthotenylbutylether sowie weitere verzweigte oder lineare, gesättigte oder ungesättigte Homologe ebenso einsetzbar. Das gleiche gilt für die Salze der Panthotensäure, deren mögliche Gegenionen nicht nur auf Calcium beschränkt sind, sondern ebenso alle physiologisch akzeptablen Metallkationen, beispielsweise die der Alkali und Erdalkalimetalle, insbesondere Magnesium, Natrium oder Kalium umfassen. Die Erfindung umfaßt auch den Einsatz aller möglichen Stereoisomeren der unterschiedlichen Vitamine, wobei insbesondere beim Vitamin B₅ und seinen Derivaten sowohl die D- als auch die L-Form und alle Mischungen der beiden Formen erfindungsgemäß eingesetzt werden können.

Bevorzugt ist der Einsatz von Vitamin C, Vitamin H, Vitamin E und seiner Derivate sowie Vitamin B₅ und seiner Derivate, wobei der Einsatz von Vitamin H, Vitamin E-Acetat, Panthenol, Panthequat® und Vitamin B₆ besonders bevorzugt ist.

In der Regel werden die Vitamine bzw. Vitamin-Derivate in den erfindungsgemäßen Zubereitungen in Mengen von etwa 0,01 bis etwa 30 Gew.-%, bezogen auf die gesamte Zubereitung, eingesetzt. Üblicherweise sind zur Herstellung wäßriger, gebrauchsfertiger Zubereitungen etwa 0,02 bis etwa 15, insbesondere etwa 0,02 bis etwa 8 Gew.-%, besonders vorteilhaft. In vielen Fälle sind Mengen zwischen etwa 0,05 und etwa 5 Gew.-% ausreichend. Für Konzentrate kann es vorteilhaft sein, Vitamine und/oder Vitaminderivate in Mengen von etwa 0,05 bis etwa 30 Gew.-%, insbesondere von etwa 1 bis etwa 25 Gew.-% und besonders bevorzugt von etwa 3 bis etwa 20 Gew.-%, einzusetzen.

Die erfindungsgemäßen Zubereitungen können neben den Komponenten A und B, alleine oder zusammen mit einem oder mehreren der bereits aufgezählten Inhaltsstoffe, auch Proteinhydrolysate enthalten. Unter Proteinhydrolysaten wird ein Gemisch aus Aminosäuren, Oligopeptiden und Polypeptiden sowie deren Derivaten verstanden.

Erfindungsgemäß einsetzbare Aminosäuren sind beispielsweise Arginin, Lysin, Cystein, Glutamin, Asparagin und Valin. Ebenfalls als geeignet haben sich Aminosäuregemische erwiesen, wie sie durch weitgehend vollständige basische, saure oder enzymatische Hydrolyse von tierischen oder pflanzlichen Proteinen erhalten werden können.

Erfindungsgemäß einsetzbare Oligopeptide und Polypeptide sind beispielsweise tierische oder pflanzliche Proteine oder deren auf saurem, basischem oder enzymatischen Wege gewonnene (Teil-)Hydrolysate. Geeignete Proteine sind beispielsweise Keratin, Kollagen, Elastin, Sojaprotein, Milchprotein, Casein, Fibroine, Sericin, Weizenprotein, Seidenprotein, Erbsenprotein und Mandelprotein. Keratin sowie die pflanzlichen Proteine erfindungsgemäß bevorzugt. Durch die Hydrolyse entstehen Stoffmischungen mit mittleren Molmassen im Bereich von etwa 400 bis etwa 50 000 Dalton. Übliche mittlere Molmassen liegen in einem Bereich von etwa 500 bis etwa 8000 Dalton. Hydrolysate von Keratin, Seidenprotein und von pflanzlichen Proteinen sind erfindungsgemäß bevorzugt.

Unter Derivaten der Aminosäuren, Oligopeptide und Polypeptide werden erfindungsgemäß deren kationische Derivate sowie deren Kondensationsprodukte mit Fettsäuren verstanden.

Kationische Derivate erhält man durch Umsetzung mit Verbindungen, die üblicherweise quartäre Ammoniumgruppen tragen oder durch Umsetzung mit entsprechenden Aminen und anschließende Quaternierung.

Eine Reihe solcher quartärer Proteinhydrolysate sind als Handelsprodukte erhältlich, beispielsweise:
- kationisches Kollagenhydrolysat, beispielsweise das unter der Bezeichnung Lamequat®L auf dem Markt befindliche Produkt (INCI-Bezeichnung: Lauryldimonium Hydroxypropyl Hydrolyzed Collagen; Chemische Fabrik Grünau),
- kationisches Keratinhydrolysat, beispielsweise das unter der Bezeichnung Croquat® auf dem Markt befindliche Produkt (CTFA-Bezeichnung: Cocodimonium Hydroxypropyl Hydrolyzed Keratin; Croda)
- kationisches Weizenhydrolysat, erhältlich unter der Bezeichnung Hydrotriticum®QL (CTFA-Bezeichnung: Lauryldimonium Hydroxypropyl Hydrolyzed Wheat Protein; Croda)
- das unter der Bezeichnung Crotein®Q erhältliche Produkt, gemäß INCI ein *"Hydroxypropyltrimonium Hydrolyzed Collagen"* (Croda) sowie
- das als Lexein®Q X 3000 (Inolex) erhältliche quatemierte Eiweißhydrolysat.

Zur Herstellung der Kondensationsprodukte von Proteinhydrolysaten mit Fettsäuren werden als Säurekomponente bevorzugt Ölsäure, Myristinsäure, Undecylensäure, Kokosfettsäure und Abietinsäure verwendet. Diese Kondensationsprodukte können auch in Form von Salzen, insbesondere Natrium-, Kalium- und Triethanolaminsalzen vorliegen.

Die Kondensationsprodukte auf Basis Kollagenhydrolysat tragen auch die CTFA-Bezeichnungen Oleoyl Hydrolyzed Collagen, Myristoyl Hydrolyzed Collagen, Oleoyl Hydrolyzed Animal Collagen, Potassium Coco Hydrolyzed Animal Protein, TEA Abietoyl Hydrolyzed Collagen, Potassium Undecylenoyl Hydrolyzed Collagen und TEA Coco Hydrolyzed Collagen. Handelsprodukte sind beispielsweise Lamepon®LPO, Lamepon®4 SK, Lamepon®UD, Lamepon®460, Lamepon®PA TR, Lamepon®ST 40 und Lamepon®S (Grünau) sowie Lexein®A 240, Lexein®S 620 und Lexein®A 520 (Inolex).

Kondensationsprodukte von Elastinhydrolysaten mit Fettsäuren wie beispielsweise Laurinsäure (CTFA-Bezeichnung: Lauroyl Hydrolyzed Elastin) können ebenfalls eingesetzt werden. Crolastin®AS (Croda) ist ein entsprechendes Marktprodukt.

Unter der Bezeichnung Promois EGCP erhältlich ist ein Potassium Cocoyl Hydrolyzed Wheat Protein (Seiwa).

Weitere erfindungsgemäß einsetzbare Handelsprodukte sind Lexein®A 200 (Inolex), Lamepon®PO-TR, Lamepon®PA-K, Lamepon®S-MV und Lamepon®S-TR (Grünau) und Crotein®CCT (Croda).

Werden die erfindungsgemäßen Zubereitungen in Haarfärbemitteln eingesetzt, so enthalten sie neben Komponente A und Komponente B weiterhin noch Färbemittel zum Färben von Keratinfasern, insbesondere sogenannte Oxidationsfärbemittel.

Da die erfindungsgemäßen Zubereitungen, enthaltend Komponente A und Komponente B, in der Regel einen pH-Wert im neutralen Bereich aufweisen, ist es möglich, unter Verwendung der erfindungsgemäßen Zubereitung Haarfärbemittel zu formulieren, deren pH-Wert ebenfalls im neutralen Bereich liegt In jedem Fall sollte der pH-Wert mindestens etwa 6 betragen. Die erfindungsgemäßen Zubereitungen sind jedoch auch in solchen Haarfärbemitteln einsetzbar, die einen pH-Wert im alkalischen Bereich, beispielsweise im Bereich von etwa 8 bis etwa 12, benötigen. Gegebenenfalls kann es dann jedoch notwendig sein, zusätzlich zur erfindungsgemäßen Kombination aus Komponente A und Komponente B eine weitere emulgierende Verbindung einzusetzen.

Oxidationsfärbemittel liegen in den erfindungsgemäßen Zubereitungen in der Regel als Oxidationsfarbstoffvorprodukte, sogenannte Entwickler und sogenannte Kuppler, vor.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2'-hydroxyethyl)-pyrazol.

Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylarninophenol, 2-Hydroxy-4-aminophenoxyethanol, 2-Methyl-5-(2-hydroxyethylamino)-phenol und 2,6-Dihydroxy-3,4-dimethylpyridin.

Die Entwickler und Kuppler werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die Haarfärbemittel enthalten sowohl die Entwickler als auch die Kuppler bevorzugt in einer Menge von etwa 0,005 bis etwa 20 Gew.-%, vorzugsweise etwa 0,1 bis etwa 5 Gew.-%, jeweils bezogen auf das gesamte Oxidationsfärbemittel. Dabei werden Entwickler und Kuppler im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwickler und Kuppler in einem Mol-Verhältnis von etwa 1 : 0,5 bis etwa 1 : 3, insbesondere etwa 1:1 bis etwa 1:2, enthalten sein können.

In einer bevorzugten Ausführungsform enthalten die Haarfärbemittel zur weiteren Modifizierung der Farbnuancen neben den Oxidationsfarbstoff-vorprodukten zusätzlich übliche direktziehende Farbstoffe, z.B. aus der Gruppe der Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Die erfindungsgemäßen Mittel gemäß die-ser Ausführungsform enthalten die direktziehenden Farbstoffe bevorzugt in einer Menge von 0,01 bis 20 Gew.-%, bezogen aufdas gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Zur Herstellung der Färbemittel werden die Oxidationsfarbstoffvorprodukte in die erfindungsgemäße Zubereitung, mindestens enthaltend Komponente A und Komponente B, eingearbeitet. Zum Zwecke der Haarfärbung sind die erfindungsgemäßen Zubereitungen z.B. als Creme, Aerosol, Emulsion, Gel oder auch als tensidhaltige, schäumende Lösungen, z.B. als Shampoo, Schaumaerosol oder in einer anderen Weise formuliert, die für die Anwendung auf dem Haar geeignet sind.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwickler oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbemittel sollte bevorzugt einen pH-Wert im Bereich von 6 bis 10 aufweisen. Besonders bevorzugt ist die Anwendung der Haarfärbemittel in einem neutralen bis schwach alkalischen Milieu. Die Anwendungstemperaturen können in einem Bereich zwischen 15 und 40°C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die Färbemittel können weiterhin alle für solche Zubereitungen bekannten Wirk-, Zusatzund Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind.

Die erfindungsgemäße Zubereitung kann ebenfalls bei der Herstellung von Wellmitteln eingesetzt werden. Unter Wellmitteln werden Mittel verstanden, die der dauerhaften Verformung von Keratinfasern dienen. Eine solche Verformung wird in der Regel dadurch erreicht, daß man die Faser mechanisch verformt und die Verformung durch geeignete Hilfmittel festlegt. Vor und/oder nach dieser Verformung behandelt man die Faser mit der wäßrigen Zubereitung einer keratinreduzierenden Substanz (Wellmittel) und spült nach einer Einwirkungszeit mit Wasser. In einem zweiten Schritt behandelt man dann die Faser mit der wäßrigen Zubereitung eines Oxidationsmittels (Fixiermittel). Nach einer Einwirkungszeit wird auch das Oxidationsmittel ausgespült und die Faser von den mechanischen Verformungshilfsmitteln (Wickler, Papilloten) befreit.

Die Wellmittel enthalten als keratinreduzierende Substanzen Mercaptane, beispielsweise Thioglykolsäure, Thiomilchsäure, Thioäpfelsäure, Mercaptoethansulfonsäure sowie deren Salze und Ester, Cysteamin, Cystein, Bunte Salze und Alkalisalze der Schwefligen Säure. Bevorzugt sind die Alkali- oder Ammoniumsalze der Thioglykolsäure und/oder der Thiomilchsäure sowie die freien Säuren. Diese werden in den Wellmitteln bevorzugt in Konzentrationen von etwa 0,5 bis 1,0 Mol/kg, bezogen auf das gesamte Wellmittel, bei einem pH-Wert von etwa 5 bis 10, insbesondere von 6,5 bis 8,5, eingesetzt.

Als Fixiermittel werden Oxidationsmittel, z. B. Natriumbromat, Kaliumbromat oder Wasserstoffperoxid, zusammen mit zur Stabilisierung wäßriger Wasserstoffperoxidzubereitungen üblichen Stabilisatoren, eingesetzt. Der pH-Wert solcher wäßriger H₂O₂-Zubereitungen, die üblicherweise etwa 0,5 bis 3,0 Gew.-% H₂O₂ enthalten, liegt bevorzugt bei 2 bis 4; er wird durch anorganische Säuren, bevorzugt Phosphorsäure, eingestellt.

Fixiermittel auf Bromat-Basis enthalten die Bromate üblicherweise in Anteilen von etwa 1 bis 10 Gew.-%, und der pH-Wert der Lösungen wird auf 4 bis 7 eingestellt Gleichfalls geeignet sind Fixiermittel auf enzymatischer Basis (Peroxidasen), die keine oder nur geringe Mengen an Oxidationsmitteln, insbesondere H₂O₂, enthalten.

Sowohl Wellmittel als auch Fixiermittel können als Creme, Gel oder Flüssigkeit formuliert sein. Weiterhin ist es möglich, die Mittel in Form von Schaumaerosolen zu konfektionieren, die mit einem verflüssigten Gas, z.B. Propan-Butan-Gemischen, Stickstoff, CO₂, Luft, N₂O, Dimethylether, Fluorchlorkohlenwasserstofftreibmitteln oder Gemischen davon in Aerosolbehältern mit Schaumventil abgefüllt werden. Die Fixiermittel können auch als Feststoffe formuliert werden. Sie enthalten das Oxidationsmittel dann in Form eines Festkörpers, z.B. Kalium- oder Natriumbromat. Erst kurz vor der Anwendung werden diese Mittel mit Wasser versetzt. Es kann ebenfalls bevorzugt sein, das Oxidationsmittel als 2-Komponenten-System zu formulieren. Die beiden Komponenten, von denen die eine bevorzugt eine Wasserstoffperoxidlösung oder eine wäßrige Lösung eines anderen Oxidationsmittels ist und die andere die übrigen Bestandteile enthält, werden ebenfalls erst kurz vor der Anwendung vermischt.

Bei den erfindungsgemäßen Haarbehandlungsmitteln handelt es sich insbesondere um Shampoos, Haarspülungen, Haarkuren, Wellotionen, Haarfärbemittel, Haarsprays, Haarwässer oder Haarspitzenfluids. Entsprechend dem Verwendungszweck können die Zubereitungen als Lösungen, Gele, Cremes, Aerosole oder Lotionen formuliert werden. Dabei kann es sich sowohl um Produkte handeln, die nach einer bestimmten Einwirkzeit, in der Regel ca. 1 bis 45 Minuten, wieder aus dem Haar ausgespült werden, als auch um Produkte die auf dem Haar verbleiben.

Entsprechend dem Verwendungszweck und der Formulierungsart können die erfindungsgemäßen Zubereitungen alle dabei üblichen kosmetischen Zusätze enthalten.

Solche üblichen Zusätze sind:
- amphotere Tenside wie N-Alkylglycine, N-Alkylpropionsäuren, N-Alkylaminobuttersäuren, N-Alkyliminodipropionsäuren, N-Hy-droxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe,
- nichtionische Polymere wie Vinylpyrrolidon/Vinylacrylat-Copolymere, Polyvinylpyrrolidon und Vinylpyrrolidon/Vinylacetat-Co-polymere,
- Verdickungsmittel wie Agar-Agar, Guar-Gum, Alginate, Celluloseether, Gelatine, Pektine und/oder Xanthan-Gum,
- Strukturanden wie Glucose und Maleinsäure,
- haarkonditionierende Verbindungen wie Phospholipide, beispielsweise Sojalecithin, Ei-Lecithin und Kephaline, sowie Silikonöle,
- Parfümöle, Dimethylisosorbit und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Climbazol, Piroctone Olamine und Zink Omadine,
- weitere Substanzen zur Einstellung des pH-Wertes,
- Wirkstoffe wie Bisabolol, Allantoin und Pflanzenextrakte,
- Lichtschutzmittel,
- Fette und Wachse, wie Walrat, Bienenwachs, Montanwachs, Paraffine, Ester, Glyceride und Fettalkohole,
- Fettsäurealkanolamide,
- Quell- und Penetrationsstoffe wie Pyrrolidoncarbonsäuren, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- Treibmittel wie Propan-Butan-Gemische, N₂O, Dimethylether, CO₂ und Luft sowie
- Antioxidantien.

Gegebenenfalls können die erfindungsgemäßen Zubereitungen auch in zweiphasigen Systemen zur Haut- oder Haarbehandlung eingesetzt werden. Ein solches Zweiphasensystem ist beispielsweise in der DE-A 195 01 188 beschrieben. Gegenstand der Erfindung ist damit auch eine Zubereitung, wie sie vorstehend beschrieben ist, die ein durch mechanische Einwirkung kurzzeitig mischbares Zwei-Phasensystem bildet.

Die erfindungsgemäße Zubereitung läßt sich bei der Herstellung einer großen Zahl von Körperpflegemitteln, insbesondere von Mitteln zur Pflege der Haut oder des Haares, insbesondere zur Pflege des Haares, einsetzen. Gegenstand der Erfindung ist damit insbesondere die Verwendung einer erfindungsgemäßen Zubereitung zur Herstellung von Haarkuren, Haarspülungen, Färbemitteln oder Wellmitteln. Ebenso ist Gegenstand der Erfindung die Verwendung einer erfindungsgemäßen Zubereitung zur Herstellung einer Hautcreme.

Ebenfalls Gegenstand der Erfindung sind damit Haarkuren, Haarspülungen, Färbemittel, Wellmittel oder Hautcremes, enthaltend eine erfindungsgemäße Zubereitung.

Die Erfindung wird nachfolgend durch Beispiele und Beispielrezepturen näher erläutert.

### BEISPIELE

### 1. Untersuchung der Avivagewirkung und der Emulgatorwirkung der erfindungsgemäßen Zubereitungen

Für die Untersuchungen diente folgende Basisformulierung, deren Mengenangaben als Gew.-% Wirksubstanz bezogen auf die gesamte Zubereitung zu verstehen sind:

| | |
|---|---|
| Stenol 1618 | 2,8 (Cetearyl Alcohol) Henkel |
| Myritol 318 | 1,0 (Caprylic/Capric Triglyceride) Henkel |
| Cutina CP | 1,20 (Cetyl Palmitate) Henkel |
| Tego Amid S 18 | 1,0 (Stearamidopropyl Dimethylamin) Goldschmid |
| | |
| Säure | q.s. |
| Foryl 100 | 0,20 (Laureth-10) |
| pHB-Methylester | 0,20 |
| pHB-Propylester | 0,20 |
| Phenoxyethanol | 0,50 |

Die zu 100 Gew.-% fehlende Menge wurde jeweils durch Wasser ergänzt.

Zur Bestimmung der Emulgatorwirkung verschiedener Säuren wurde die o.g. Basisformulierung vorgelegt und mit einer der ausgewählten Säuren versetzt. Es wurde jeweils soviel Säure zugegeben, bis eine Emulsion entstand und anschließend der pH-Wert der Emulsion gemessen (Ausnahme : Versuch 2). In Versuch 2 wurde angestrebt, mit einer nicht erfindungsgemäßen Säure eine Emulsion mit einem möglichst nahe am Neutralpunkt liegenden pH-Wert herzustellen.

Folgende Säuren wurden getestet (Tabelle 1):

**Tabelle 1**

| (Versuch) Säure | Menge in Gew.-% | Viskosität [mPas] | pH-Wert |
|---|---|---|---|
| Vergleichsbeispiel (1) Citronensäure w-frei | 0,25 | 8000 | 4,25 |
| Vergleichsbeispiel (2) Citronensäwe w-frei | 0,17 | keine Emulsion | 5,57 |
| Vergleichsbeispiel (3) Milchsäure 90%ig | 0,27 | <1000 | 5,60 |
| Vergleichsbeispiel (4) Bernsteinsäure | 0,16 | 9000 | 4,71 |
| (5) EDTA | 0,20 | 6400 sehr weiß | 7,25 |
| (6)NTA | 0,17 | 5300 | 6,91 |
| (7) EDETA GS Beta-Alanine Diacetic Acid (BASF) | 0,18 | >10000 | 6,90 |

Die Ergebnisse in Tabelle 1 zeigen, daß es nur unter den erfindungsgemäßen Bedingungen möglich ist, Emulsionen mit einem im wesentlichen neutralen pH-Wert zu erhalten.

## Patentansprüche

1. Wäßrige Zubereitung zur Haut- oder Faserbehandlung, enthaltend mindestens ein Alkylamidoamin der allgemeinen Formel I 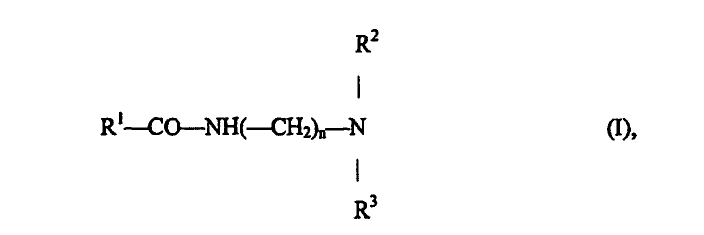 worin R¹-CO für einen aliphatischen, linearen oder verzweigten, bis zu 3 Doppelbindungen aufweisenden C₆-C₂₂-Acylrest steht, n Werte von 1 bis 10 bedeutet und R² und R³ unabhängig voneinander für lineare oder verzweigte C₁-C₂₂-Alkylreste stehen, **dadurch gekennzeichnet, daß** sie zusätzlich mindestens eine, mindestens eine primäre, sekundäre oder tertiäre Aminogruppen aufweisende Polycarbonsäure enthält und das molare Verhältnis von Alkylamidoaminmolekülen zu freien Säuregruppen zwischen 0,9 und 1,1 liegt.

2. Zubereitung nach Anspruch 1, **dadurch gekennzeichnet, daß** der pH-Wert 6,5 bis 7,5 beträgt.

3. Zubereitung nach Anspruch 1 oder 2, **dadurch gekennzeichnet, daß** die mindestens eine Polycarbonsäure ein Derivat einer Di-, Tri- oder Tetracarbonsäure ist.

4. Zubereitung nach einem der vorstehenden Ansprüche, **dadurch gekennzeichnet, daß** n für Werte von 2 bis 6 steht

5. Zubereitung nach einem der vorstehenden Ansprüche, **dadurch gekennzeichnet, daß** R¹-CO für einen unverzweigten C₁₄-C₂₀-Acylrest steht.

6. Zubereitung nach einem der vorstehenden Ansprüche, **dadurch gekennzeichnet, daß** R² und R³ unabhängig voneinander für C₁-C₆-Alkylreste stehen.

7. Zubereitung nach einem der vorstehenden Ansprüche, **dadurch gekennzeichnet, daß** als mindestens eine Polycarbonsäure Nitrilotriessigsäure (NTA), Ethylendiamintetraessigsäure (EDTA), β-Alanindiessigsäure (ADA), Asparaginsäure (ASP) oder Glutaminsäure (GLU) enthalten ist.

8. Zubereitung nach einem der vorstehenden Ansprüche, **dadurch gekennzeichnet, daß** sie noch mindestens eine Verbindung, ausgewählt aus der Gruppe der kationischen Polymere, anionischen Polymere, amphoteren Polymere, kationischen Tenside, kationischen Silikonöle, quaternären Esterverbindungen, anionischen Tenside, nichtionischen Tenside, Fettalkohole, Vitamine oder Vitaminderivate, Färbemittel, Lösemittel und Konservierungsmittel.

9. Zubereitung nach einem der vorstehenden Ansprüche, **dadurch gekennzeichnet, daß** sie ein durch mechanische Einwirkung kurzzeitig mischbares 2-Phasensystem bildet.

10. Verwendung einer Zubereitung gemäß einem der vorstehenden Ansprüche zur Herstellung von Haarkuren, Haarspülungen, Färbemitteln oder Wellmitteln.

11. Verwendung einer Zubereitung gemäß einem der Ansprüche 1 bis 9 zur Herstellung einer Hautcreme.

12. Haarkur, Haarspülung, Färbemittel, Wellmittel oder Hautcreme, enthaltend eine Zubereitung gemäß einem der Ansprüche 1 bis 9.

## Claims

1. A water-based preparation for treating the skin or fibres containing at least one alkyl amidoamine corresponding to general formula I: 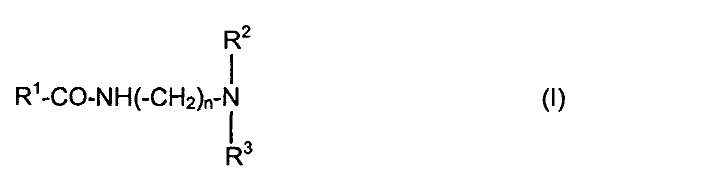 in which R¹-CO is an aliphatic, linear or branched C₆₋₂₂ acyl group containing up to 3 double bonds, n has a value of 1 to 10 and R² and R³ independently of one another represent linear or branched C₁₋₂₂ alkyl groups, **characterized in that** it additionally contains at least one polycarboxylic acid containing at least one primary, secondary or tertiary amino group and the molar ratio of alkyl amidoamine molecules to free acid groups is between 0.9 and 1.1:1.

2. A preparation as claimed in claim 1, **characterized in that** it has a pH of 6.5 to 7.5.

3. A preparation as claimed in claim 1 or 2, **characterized in that** the at least one polycarboxylic acid is a derivative of a di-, tri- or tetracarboxylic acid,

4. A preparation as claimed in any of the preceding claims, **characterized in that** n has a value of 2 to 6.

5. A preparation as claimed in any of the preceding claims, **characterized in that** R¹-CO stands for an unbranched C₁₄₋₂₀ acyl group.

6. A preparation as claimed in any of the preceding claims, **characterized in that** R² and R³ independently of one another are C₁₋₆ alkyl groups.

7. A preparation as claimed in any of the preceding claims, **characterized in that** it contains nitrilotriacetic acid (NTA), ethylenediamine tetraacetic acid (EDTA), β-alanine diacetic acid (ADA), aspartic acid (ASP) or glutamic acid (GLU) as at least one polycarboxylic acid.

8. A preparation as claimed in any of the preceding claims, **characterized in that** it additionally contains a compound selected from the group of cationic polymers, anionic polymers, amphoteric polymers, cationic surfactants, cationic silicone oils, quaternary ester compounds, anionic surfactants, nonionic surfactants, fatty alcohols, vitamins or vitamin derivatives, colorants, solvents or preservatives.

9. A preparation as claimed in any of the preceding claims, **characterized in that** it forms a two-phase system which can be mixed in a short time by mechanical action.

10. The use of the preparation claimed in any of the preceding claims for the production of hair treatments, hair rinses, colorants or wave sets.

11. The use of the preparation claimed in any of claims 1 to 9 for the production of a skin cream.

12. A hair treatment, hair rinse, colorant, wave set or skin cream containing the preparation claimed in any of claims 1 to 9.

## Revendications

1. Préparation aqueuse pour traiter la peau ou les fibres, contenar au moins une alkylamidoamine de formule générale I 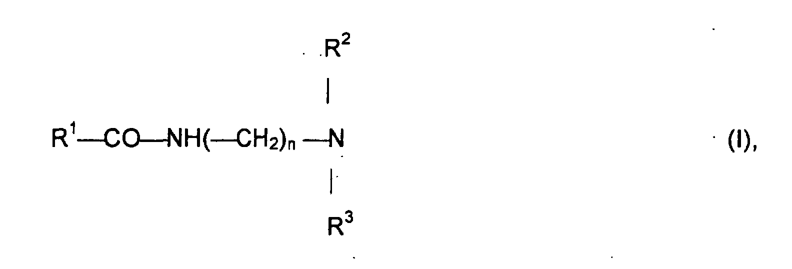 dans laquelle R¹-CO représente un résidu acyle en C₆ à C₂₂, aliphatique, linéaire ou ramifié, présentant jusqu'à 3 doubles liaisons, n représente des valeurs de 1 à 10, et R² et R³ représentent indépendamment l'un de l'autre des résidus alkyle en C₁ à C₂₂, linéaires ou ramifiés, **caractérisée en ce qu'**elle contient en outre au moins, un poly(acide carboxylique) présentant au moins un groupe amine primaire, secondaire ou tertiaire, et que le rapport molaire des molécules d'alkylamidoamine au groupe acide libre est compris entre 0,9 et 1,1.

2. Préparation selon la revendication 1, **caractérisée en ce que** la valeur du pH est de 6,5 à 7,5.

3. Préparation selon la revendication 1 ou 2, **caractérisée en ce que** ledit au moins un poly(acide carboxylique) est un dérivé d'un acide di-, tri- ou tétra-carboxylique.

4. Préparation selon l'une quelconque des revendications précédentes, **caractérisée en ce que** n représente des valeurs de 2 à 6.

5. Préparation selon l'une quelconque des revendications précédentes, **caractérisée en ce que** R¹-CO représente un résidu acyle en C₁₄ à C₂₀, non ramifié.

6. Préparation selon l'une quelconque des revendications précédentes, **caractérisée en ce que** R² et R³ représentent indépendamment l'un de l'autre, des résidus alkyle en C₁ à C₆.

7. Préparation selon l'une quelconque des revendications précédentes, **caractérisée en ce que** l'acide nitrilotriacétique (NTA), l'acide éthylènediamine-tétraacétique (EDTA), l'acide β-alanine-diacétique (ADA), l'acide aspartique (ASP) ou l'acide glutamique (GLU) y est contenu en tant qu'au moins un poly(acide carboxylique).

8. Préparation selon l'une quelconque des revendications précédentes, **caractérisée en ce qu'**elle contient encore au moins un composé, choisi dans le groupe formé par les polymères cationiques, les polymères anioniques, les polymères amphotères, les tensio-actifs cationiques, les huiles siliconées cationiques, les composés ester quaternaires, les tensio-actifs anioniques, les tensio-actifs non ioniques, les alcools gras, les vitamines ou les dérivés des vitamines, les colorants, les solvants et les conservateurs.

9. Préparation selon l'une quelconque des revendications précédentes, **caractérisée en ce qu'**elle forme un système biphasique miscible à brève échéance au moyen d'une action mécanique.

10. Utilisation d'une préparation selon l'une quelconque des revendications précédentes, pour la préparation de cures capillaires, de rinçages capillaires, de colorants ou d'agents de permanente.

11. Utilisation d'une préparation selon l'une quelconque des revendications 1 à 9, pour préparer une crème pour la peau.

12. Cure capillaire, rinçage capillaire, colorant, agent de permanente ou crème pour la peau, contenant une préparation selon l'une quelconque des revendications 1 à 9.
